# EUROPEAN PATENT APPLICATION

(11) **EP 4 742 255 A1**
(43) Date of publication of application: **13.05.2026**
(21) Application number: 25215005.7
(22) Date of filing: 11.11.2025
(51) Int. Cl.: G16H 10/20, G16H 10/60, G16H 40/20

(54) **SYSTEMS AND METHODS FOR AUTONOMOUS REAL-TIME GENERATION AND MODIFICATION OF A DYNAMIC ELECTRONIC TRIAL MASTER FILE**

(30) Priority: 12.11.2024 US 202463719443 P; 24.10.2025 US 202519368977
(71) Applicant: Medidata Solutions, Inc., New York, NY 10014 (US)
(72) Inventor: FRID-NIELSEN, Martin, New York 10014 (US); CHAN, Yau Pan, New York 10014 (US); GLAVIN, Timothy, New York 10014 (US); RICHMOND, Kately, New York 10014 (US); NANDI, Umamaheswara, New York 10014 (US)
(74) Representative: D Young & Co LLP

(57) **Abstract**

Relates to autonomous real-time generation and modification of a dynamic hierarchical file structure for an electronic Trial Master File (eTMF). A method includes receiving, by an event processor, event messages from a message system integrating a plurality of applications, the applications configured to automatically generate messages in real time in response to events. The method further includes determining, by the event processor, for each event message, one or more hierarchical file structure placeholders associated with the corresponding activity based on the metadata of each said event message. The method further includes automatically modifying in real time, by the event processor, the hierarchical file structure to incorporate said one or more hierarchical file structure placeholders. The method further includes storing one or more documents received from the computer system at the first site in said one or more hierarchical file structure placeholders.

## Description

### FIELD

The present disclosure generally relates to autonomous real-time generation and modification of a dynamic electronic Trial Master File.

### BACKGROUND

The electronic Trial Master File (eTMF) serves as the central repository for various documents collected throughout the duration of a clinical trial. Regulatory bodies, such as the U.S. Food and Drug Administration (FDA) and their counterparts in other countries, frequently inspect the eTMF to ensure that the clinical trial is being conducted in a compliant and regulated manner.

Conventional approaches to creating an eTMF are predominantly manual and labor-intensive. Typically, these processes begin with defining the structure of the eTMF. This involves generating a file plan that outlines the relevant document types required for the trial. Teams then manually decide on the appropriate documentation based on the study's nature and create a hierarchical structure populated with placeholders for these documents.

Such methods necessitate significant manual intervention, including the printing and scanning of documents. Once printed and filled out by hand, documents are scanned back into the system, reviewed, and then placed in the correct location within the eTMF's folder structure. This process is repeated for each document, leading to a highly time-consuming and inefficient workflow. As further example, ongoing quality control reviews are needed as regulations and/or the study changes over time (e.g., the addition or removal of sites).

In conventional approaches, a file plan is created based on industry-standard reference models, e.g., the Clinical Data Interchange Standards Consortium (CDISC) Trial Master File Reference Model, which is the current standard in the field (the "TMF Reference Model"). This plan dictates the structure and types of documents needed, which are then manually inputted into the eTMF. Despite advancements in digital technologies, a substantial amount of paper is still used, as documents are often printed, filled out manually, and then scanned back into the system. Thus, overall, the current state of eTMF management is characterized by its manual nature, requiring significant human effort to review, categorize, and file documents appropriately.

### SUMMARY

Particular aspects are set out in the appended independent claims. Various optional embodiments are set out in the dependent claims.

In view of the deficiencies in conventional approaches discussed above, it is imperative to provide a technical solution to the technical problem of providing more efficient, automated solutions to streamline eTMF processes and enhance the productivity of clinical trial documentation management.

Disclosed embodiments provide a system, computer-implemented method, and computer-readable medium for autonomous real-time generation and modification of a dynamic hierarchical file structure for an electronic Trial Master File (eTMF), the method includes receiving, by an event processor, event messages from a message system integrating a plurality of applications, the applications configured to automatically generate messages in real time in response to events. Each of the events corresponds to a user interaction with one of the applications via a user interface of a computer system at a first site based on an activity associated with the clinical trial, and each event message corresponding to each said user interaction comprises metadata relating to a corresponding activity. The method further includes determining, by the event processor, for each event message, one or more hierarchical file structure placeholders associated with the corresponding activity based on the metadata of each said event message. The method further includes automatically modifying in real time, by the event processor, the hierarchical file structure to incorporate said one or more hierarchical file structure placeholders. The method further includes storing one or more documents received from the computer system at the first site in said one or more hierarchical file structure placeholders.

Embodiments may include one or more of the following features.

The metadata relating to the corresponding activity may include one or more of: a site identifier associated with the site, a milestone identifier corresponding to the activity, and a role designation for a user associated with the activity. The method may include notifying, in real time, one or more users at the first site as placeholders are created in the hierarchical file structure. The method may include automatically validating the one or more documents received from the computer system against predefined formats or compliance criteria before storing them in the hierarchical file structure placeholders.

The determining, by the event processor, of the one or more hierarchical file structure placeholders may include dynamically applying one or more predefined templates from a template library, the templates specifying expected document types for activities associated with the clinical trial. The automatically modifying in real time of the hierarchical file structure may include dynamically adapting the structure to fulfill region-specific requirements associated with the first site.

The method may include tracking the completeness of the hierarchical file structure placeholders across the plurality of sites, and generating real-time quantitative metrics for placeholders that are complete or pending at each site. The method may include generating alerts when one or more placeholders remain unfulfilled beyond a predetermined compliance deadline, wherein the alerts are sent to designated users. The method may include automatically replacing existing placeholders in the hierarchical file structure with updated placeholders in response to receiving event messages indicating changes to corresponding activities.

The hierarchical file structure placeholders may be organized into packages, each package being associated with a particular milestone or activity in the clinical trial. The method may include associating the one or more documents with placeholder-specific metadata, wherein the metadata includes document type, author, version, and applicable milestone identifier. The hierarchical file structure may be configured to logically separate placeholders into zones and sections corresponding to study phases, trial sites, or geographic regions.

Disclosed embodiments further provide a system for autonomously creating and managing a dynamic electronic Trial Master File (eTMF) in real time for clinical trials. The system includes a plurality of integrated applications configured to manage various aspects of the clinical trial and generate signals based on clinical trial activities. The system further includes an event processor configured to receive and process the signals to create and dynamically update the eTMF in real time. The system further includes a document repository configured to store and manage documents across multiple studies. The system further includes a document generation module configured to use templates and contextual data to generate documents and populate the dynamic eTMF in real time. The system further includes a large language model (LLM) configured to interpret signals and assist in the automatic generation and organization of the dynamic eTMF. The system further includes an integration module configured to retrieve necessary documents from external and internal repositories.

Embodiments may include one or more of the following features.

A user interface may be configured to prompt users for ad hoc document uploads when required. The event processor may dynamically update the eTMF in real time based on the received signals. The signals may be in the form of messages sent via a message system connecting the integrated applications. The system may include a module configured to group placeholders into packages, each package comprising a collection of placeholders required for specific scenarios within the clinical trial. The document generation module may use predefined templates to dynamically create documents based on contextual data. The document repository may include advanced data security measures such as encryption, access controls, and audit trails. The large language model (LLM) may continuously learn and improve its processes based on the data it processes and feedback received. The integration module may support seamless data flow and real-time updates across different components of the clinical trial process. The placeholders and generated documents may be organized in a structured and logical manner to ensure regulatory compliance and audit readiness and to comply with the organization of the reference model.

Disclosed embodiments provide a method for autonomously creating and managing an electronic Trial Master File (eTMF) for clinical trials. The method includes integrating multiple applications to manage various aspects of the clinical trial and to generate and receive signals based on clinical trial activities. The method further includes receiving and processing the signals to automatically generate and dynamically update the dynamic eTMF in real time. The method further includes generating documents using predefined templates and contextual data to populate the dynamic eTMF. The method further includes interpreting signals and assisting in the automatic generation and organization of the dynamic eTMF using a large language model (LLM). The method further includes dynamically updating the eTMF in real time based on the received signals.

Embodiments may include one or more of the following features.

The method may include prompting users for ad hoc document uploads when required. The method may further include grouping placeholders into packages, each package comprising a collection of placeholders required for specific scenarios within the clinical trial. The method may further include implementing advanced data security measures such as encryption, access controls, and audit trails in the document repository. The method may further include continuously learning and improving the processes of the LLM based on the data it processes and feedback received. The method may further include storing and managing documents across multiple studies in a document repository. The method may further include retrieving necessary documents from external and internal repositories.

Disclosed embodiments may include creating an eTMF structure with placeholders for artifacts based on received events (e.g., in the form of signals or event messages) and/or existing file plans. Placeholders in an eTMF structure may be populated based on analyzing key documents such as the Study Protocol, FDA Form 1572 and Clinical Trial Agreement, etc. Content in the dynamic eTMF structure (e.g., e-Learning certificates) may be created and automatically filed in real time based on incoming signals (e.g., event messages). Content in the dynamic eTMF structure (e.g., the Master File Note list) may be created and automatically filed in real time based on content in other artifacts already filed in the eTMF data store. Configuration of the functionality of an application, e.g., the Site Monitoring application, may be automated based on artifact content filed in the eTMF data store, such as the Monitoring Plan.

Disclosed embodiments provide a computer-readable medium comprising (e.g. storing and/or conveying) instructions that, when executed by a processor, cause the processor to perform the methods described herein.

### BRIEF DESCRIPTION OF THE DRAWINGS

Fig. 1 is a diagram of a system for autonomous real-time generation and modification of a dynamic electronic Trial Master File, according to disclosed embodiments.
Fig. 2 is a table showing examples of the results of using machine learning algorithms to analyze documents.
Fig. 3 depicts an event handling definition screen to allow input of parameters to be used in event processing.
Fig. 4 is a sequence diagram depicting messaging for a Visit Scheduled event.
Figs. 5-7 are schemas for use by the Event Processor to perform event handling via the message system.
Fig. 8 is a list of examples of message system events (or "topics") which are used by the Event Processor in event handling.
Fig. 9 is a flowchart of a method for autonomous real-time generation and modification of a dynamic electronic Trial Master File, according to disclosed embodiments.

Where considered appropriate, reference numerals may be repeated among the drawings to indicate corresponding or analogous elements. Moreover, some of the blocks depicted in the drawings may be combined into a single function.

### DETAILED DESCRIPTION

In the following detailed description, numerous specific details are set forth in order to provide a thorough understanding of embodiments of the presently taught approaches. However, it will be understood by those of ordinary skill in the art that the embodiments of the presently taught approaches may be practiced without these specific details. In other instances, well-known methods, procedures, components, and circuits have not been described in detail so as not to obscure the presently taught approaches.

Every clinical study uses an electronic Trial Master File (eTMF) as the official documentation of the clinical study. In general, the eTMF is a compilation of essential documents that are collected as a clinical trial progresses to facilitate the operation of the clinical trial and to document regulatory compliance. A file plan enables one to specify the relevant document types (i.e., "artifacts") that users should provide at each stage of the clinical trial. The file plan also enables one to define a hierarchical file structure and metadata for the documents at specific levels in the eTMF structure, e.g., product, trial, country, and site levels. At the site level, for example, a medical license needs to be collected for the Principal Investigator (PI) at each site participating in a clinical study. The hierarchical file structure and the document "placeholders" for all active stages within the file structure are created based on the file plan. Contributors and authors create and import documents and associate them with the placeholders. The eTMF system names, files, and secures the documents.

To keep track of the completeness of the eTMF, placeholders are often used to indicate documents to be collected. In general, "completeness" may be defined as the ratio between placeholders and completed documents (i.e., documents which have been uploaded and stored in the eTMF). There is often a need to collect different documents, according to a reference model, for different studies, countries, and sites depending on the phase of the study, the type of study (e.g., interventional or observational, drug or device), regulatory requirements, and the risk level of the study.

Typically, it takes weeks, or months, to get an eTMF configured and may require significant support from a vendor. Once the eTMF is configured, there is ongoing manual work to keep the, in essence, static eTMF current and updated during the execution of the clinical study. For example, sites may be added or removed and Adverse Events (AEs) may occur that require documentation in the eTMF.

The embodiments disclosed herein introduce a novel approach to eTMFs for clinical trials. Unlike conventional methods, which are largely static, manual, and labor-intensive, the disclosed approaches provide a dynamic eTMF system which is configured to create and populate itself autonomously using various sources of signals and data. This may begin with the creation of placeholders within the dynamic eTMF structure, which are subsequently filled with actual documents. By leveraging the system's data and signals (e.g., in the form of messages), the dynamic eTMF can autonomously create placeholders and generate (and/or receive) and organize these documents in real time, reducing the need for manual input and oversight. For example, upon initiating a clinical trial, the system can automatically identify and create placeholders for essential documents, such as the study protocol, based on predefined criteria and signals.

This autonomous, dynamic approach aims to simplify the eTMF creation process, making it much easier and more convenient for users. Instead of requiring detailed demonstrations and extensive manual setup, the system can be provided to users as a ready-to-use solution. This would significantly streamline the onboarding process for new studies, allowing users to focus on their core activities rather than the administrative burden of setting up and managing the eTMF.

With initial data points, such as the trial phase and whether it involves a medical device, the system can start generating the eTMF automatically. Additional signals can be gathered in real time to dynamically refine and build out the eTMF structure. Users may also be prompted to provide specific documents, such as the study protocol, which contains important information about the study's execution, participant criteria, and other procedural details. By incorporating this information, the system can automate further aspects of the dynamic eTMF management, ensuring a comprehensive and organized repository of trial documents.

The system also integrates various applications, including e-consent, adverse events, and study management, into a cohesive framework. Each application generates signals and data that are processed to automatically and dynamically populate the eTMF in real time. This integration allows for seamless data flow and real-time updates across different components of the clinical trial process.

In addition, the system employs large language models (LLMs) to interpret complex data inputs and generate the necessary documentation structure. LLMs process signals and data to create artifacts, ensuring that the dynamic eTMF is comprehensive and accurately reflects the trial's requirements in real time. The use of LLMs for data interpretation and document generation is a sophisticated method to extract data relevant for clinical trials from a variety of data sources.

The system also includes a centralized document repository that allows documents to be stored once and reused across multiple studies. This repository ensures that documents such as medical licenses are always up-to-date and can be referenced in any relevant study, reducing redundancy and optimizing storage.

Overall, the autonomous eTMF generation and modification system provides a robust and innovative technical solution to the challenges of managing clinical trial documentation. Through the use of advanced automation techniques, integration of diverse applications, utilization of large language models, event-driven processing, and contextual analysis, the system offers specific technical improvements that enhance the efficiency, accuracy, and reliability of eTMF management.

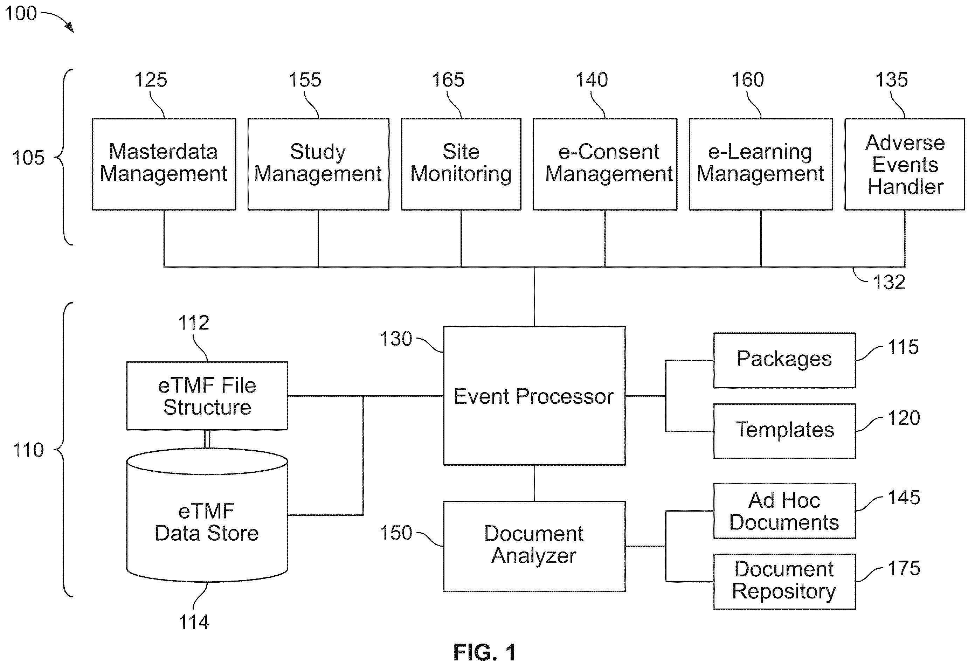

Figure 1 is a diagram of a system 100, referred to as an autonomous eTMF generation and modification system, according to disclosed embodiments. The system architecture for the autonomous eTMF generation and modification system 100 includes various components and modules designed to automate the creation and dynamic real-time management of the eTMF. In the example depicted, the system 100 has two major components: the application layer 105 and the dynamic eTMF system 110.

The application layer 105 represents various applications and services that contribute information and data to feed into the dynamic eTMF system 110 in real time, which includes the eTMF itself and other components which are discussed in greater detail below. As the system 100 receives this information in real time, it continuously and automatically processes the information and determines its impact on the structure and content of the dynamic eTMF system 110. In embodiments, the dynamic eTMF itself may include an eTMF file structure 112 and an eTMF data store 114. The application layer 105 provides information, through "events," which the dynamic eTMF system 110 can act upon.
An "event" refers to a user-initiated interaction with a user interface on a computer system, occurring at a clinical trial site or related entity, where the interaction is performed in response to a real-world activity or milestone associated with the clinical trial. This interaction generates an electronic signal, message, or notification-referred to as the "event message"-which encapsulates metadata related to the real-world activity or milestone and is transmitted to the Event Processor for further automated processing. An "event" may correspond to, but is not limited to, activities such as the confirmation of a site visit, the submission or approval of a document, the creation of a participant record, or the acknowledgment of a regulatory milestone. While the event may derive from a real-world activity (e.g., completing a site visit, screening a patient), the term specifically refers to the computer-mediated interaction that records, processes, and transmits data resulting from the user's action.

In addition to the event itself, there is also contextual information that allows the system 100 to take action, such as using an inventory of predefined data in the form of packages 115 and templates 120 as inputs for the dynamic eTMF system 110. The autonomous eTMF generation and modification system 100 effectively integrates the various applications and associated use cases to automate and streamline the collection and management of clinical trial information. Each application plays a specific role in the clinical trial process, and contributes data to the dynamic eTMF system 110.

These applications contain the necessary information for the dynamic eTMF system 110 in different formats and through various mechanisms. For example, some data is stored in databases, while other information might be available as files that can be directly retrieved. The challenge lies in having the system 100 efficiently extract and integrate this information into the dynamic eTMF system 110. By using innovative methods and mechanisms to generalize and streamline these processes, the system 100 can help ensure that all required data is accurately and efficiently collected.

The disclosed approaches seek to create a highly efficient and automated system where each application can seamlessly participate in the building and management of the dynamic eTMF system 110. This involves designing a streamlined process that facilitates easy integration and data retrieval from the various applications. By doing so, the system can minimize manual intervention, reduce errors, and help ensure that the eTMF is comprehensive and up-to-date.

### Masterdata Management Application

One of the applications in the application layer 105 is the Masterdata Management application 125, which handles the overarching definition of the study, including the sites, and countries, where the study is conducted. It also manages the users associated with the study, both at the site level and the sponsor level. The Masterdata Management application 125 generates signals such as study creation, site addition or removal, and user addition or removal. These signals are important for maintaining an up-to-date and accurate eTMF structure.

For example, using the functions of the Masterdata Management application 125, a site team can be defined. For example, assume that a new Clinical Research Coordinator is added to the study. In such a case, the resume of this person must be provided to the dynamic eTMF system 110, and possibly other types of data, such as a medical license. When the Masterdata Management application 125 notifies the dynamic eTMF system 110 about this addition (and provides information about the associated study and site as context), the dynamic eTMF system 110 can access a predefined package 115 that specifies which document placeholders are required. In embodiments, these packages 115 include contextual information, because, for example, a different set of placeholders may be needed for particular sites in particular countries. The Event Processor 130 matches the incoming event information with the package metadata to determine which package to apply.

In embodiments, as new clinical trial sites are added or removed, or as users join or leave the study, the Masterdata Management application 125 automatically dynamically updates the eTMF (via the dynamic eTMF system 110) to reflect these changes. This automation ensures that the eTMF remains a reliable and comprehensive repository of all trial-related documents.

The Masterdata Management application 125 is just one example of the many components that contribute to the autonomous eTMF generation and modification system 100. By integrating these applications, the system 100 can automatically generate and organize necessary documentation, significantly reducing manual intervention and enhancing the efficiency and accuracy of clinical trial management. This approach not only ensures regulatory compliance but also streamlines the entire process, making it more user-friendly and efficient.

### e-Learning Management Application

Another application in the application layer 105 is the e-Learning Management application 160. In a clinical study, all personnel at the site level must undergo specific training. The e-Learning Management application 160 generates signals indicating when training is due for these individuals and sends them to the Event Processor 130, prompting the creation of placeholders for the corresponding training certificates. Once the training is completed, the system 100 can use a combination of signals and APIs to obtain the actual training certificates. These certificates are then filed in the appropriate placeholders, marking them as satisfied.

The process of satisfying placeholders with actual documents is integral to maintaining the completeness of the eTMF. For example, when a user completes their training, the Event Processor 130 must retrieve the training certificate. The system 100 is designed to understand how to handle various events, knowing what actions to take to obtain the necessary documents. Once the document is filed in the placeholder, the placeholder is marked as complete, ensuring that the eTMF is up-to-date and comprehensive. The system's use of placeholders and automated document handling, as exemplified by the e-Learning Management application 160, illustrates a key aspect of the autonomous eTMF generation and modification system 100.

### Adverse Events Handler

Another application in the application layer 105 is the Adverse Events Handler 135. This application documents and manages any adverse events (or safety events) that occur during the clinical trial. When an adverse event occurs, such as a contamination or a need for clarification, it is necessary to inform all relevant parties and initiate the necessary processes to address the issue. The Adverse Events Handler 135 ensures that these adverse events are thoroughly documented and communicated. This may include detailing the nature of the event, the steps taken to mitigate it, and the communications sent to other sites. Adverse events are an important component of a clinical study, as they provide insights into potential risks and ensure that the study adheres to regulatory requirements for safety and reporting.

For example, if a participant experiences an unexpected illness due to the medication being tested, the Adverse Events Handler 135 will document all necessary details. This documentation is necessary for regulatory compliance, as it allows entities like the FDA to review how such incidents were mitigated and communicated. The system 100 helps ensure that the information generated by this module is properly, and automatically, added to the eTMF via the dynamic eTMF system 110. It is also important to record that the documentation has been distributed to all relevant sites. In embodiments, the system 100 may automate distribution of a single artifact (e.g., an adverse event report) from the site reporting the safety event to the other sites in the particular study.

### e-Consent Management Application

Another application in the application layer 105 is the e-Consent Management application 140, which manages patient consent forms. This application generates signals and information that can be processed by the Event Processor 130 to automatically feed into the dynamic eTMF system 110.

### Ad Hoc Documents Handler

The system 100 includes an Ad Hoc Documents Handler 145 for receiving ad hoc documents. For example, users might be prompted to upload the study protocol. This document is then analyzed using machine learning algorithms to extract the necessary information, which is subsequently used to populate the relevant sections of the eTMF. This automated analysis and population process reduces the need for manual data entry and ensures that the eTMF is consistently up-to-date with accurate information. In embodiments, a user interface may be configured to prompt users for ad hoc document uploads when specific information is required but not automatically generated by the system. This proactive approach ensures that all necessary documentation is collected, even if it requires manual input from users.

In embodiments, when the study protocol becomes available, either through an application that provides event information about its availability or by directly bringing it into the dynamic eTMF system 110 (e.g., via the Ad Hoc Documents Handler 145), machine learning algorithms in the Document Analyzer 150 can be used to extract information about the trial, such as the phase of the trial. This extracted information is then sent to the Event Processor 130, where it is used to determine, for example, whether placeholders for safety reporting and regulatory approvals are needed in the eTMF. In some cases, the Event Processor 130 may also send events (e.g., in the form of signals or messages) based on the extracted information to one or more of the applications in the application layer 105. For example, information, such as the phase of the trial, may be sent to the e-Learning Management application 160 to facilitate the implementation of the required training. In embodiments, the extracted information may be sent to external applications and/or systems. For example, information extracted from the "Schedule of Activities" portion of the study protocol may be sent to an Electronic Data Capture (EDC) application.

As discussed above, the system 100 uses packages 115 and templates 120 to structure and manage the eTMF efficiently. A package 115 can be described as a collection of placeholders. These placeholders are predefined slots within the eTMF that indicate where specific documents need to be placed. For example, a package 115 might be created to include placeholders for documents required when a new study site is added. This package might encompass placeholders for various documents associated with the new site.

Similarly, another package 115 could be designed for scenarios where a new principal investigator is assigned to a site. This package would include placeholders for the necessary documents related to the new investigator. By grouping these placeholders into packages, the system ensures that all required documents are systematically organized and easily accessible within the eTMF.

Templates play an important role when generating content for the eTMF. In cases where documents need to be created based on stored data, templates are used to format and structure this information. For instance, in the context of e-learning, if the data indicates that a user completed training, a template can be used to generate a certificate or other relevant document. The template combines with the Event Processor 130 to create the actual document, such as a PDF, which is then filed in the appropriate placeholder within the eTMF.

### Document Repository

In embodiments, the system 100 may include a document repository 175 for the management and storage of documents. The document repository 175 is used to maintain a centralized storage system that can be leveraged across multiple studies. This ensures that certain documents, which are frequently used in different studies, are stored once and reused as needed, thereby enhancing efficiency and consistency.

For example, consider a hospital conducting multiple clinical studies with the same Principal Investigator. The medical license of this doctor remains constant across these studies. By maintaining the doctor's medical license in the document repository, the system can easily access and utilize this document for any study involving this investigator. This avoids the redundancy of repeatedly collecting the same document for each new study.

In embodiments, the document repository 175 can connect to both external and internal repositories, enabling integration and data retrieval from various sources. This integration allows the system to automatically pick up necessary documents from these repositories, further reducing the need for manual intervention. External repositories might include regulatory databases or other institutional repositories, while internal repositories could be part of the organization's document management system.

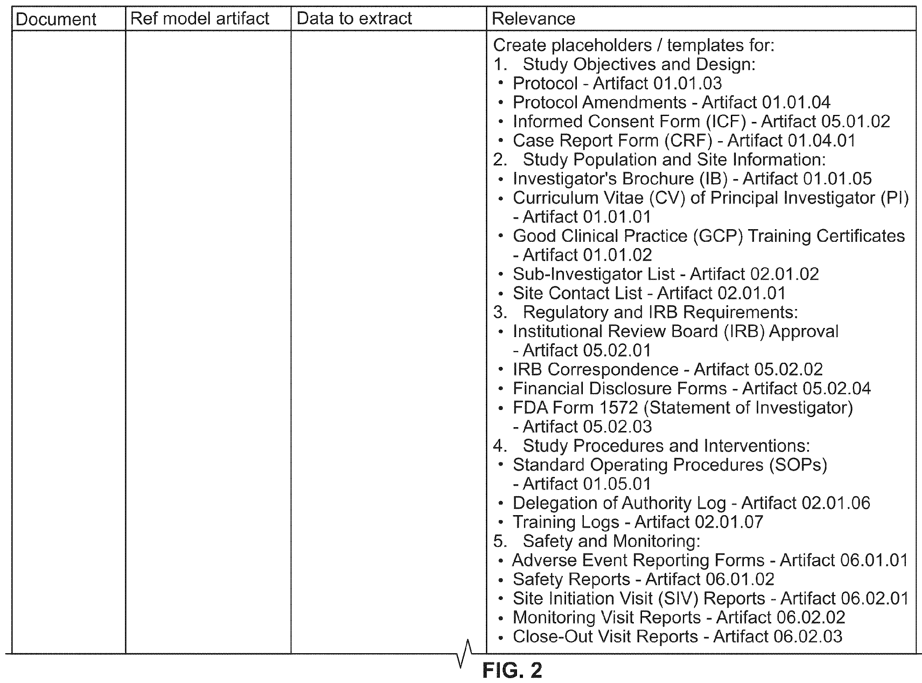

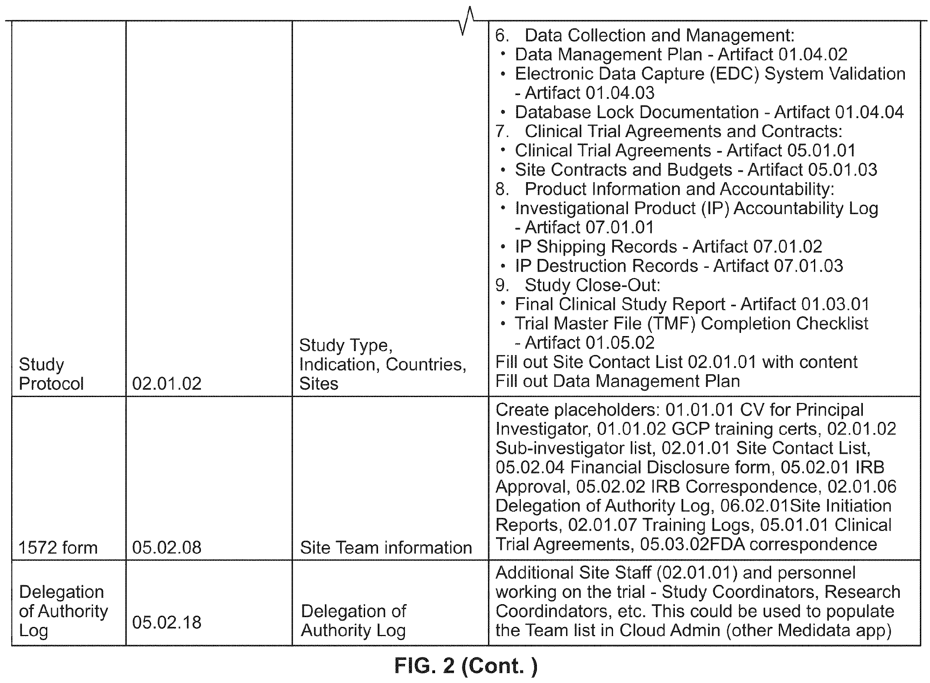

Figure 2 is a table showing examples of the results of using machine learning algorithms to analyze documents. In this example, analysis of the study protocol document provides a number of potential placeholders for the eTMF, such as: Protocol - Artifact 01.01.03; Protocol Amendments - Artifact 01.01.04; Informed Consent Form (ICF) - Artifact 05.01.02; Case Report Form (CRF) - Artifact 01.04.01; FDA Form 1572 (Statement of Investigator) - Artifact 05.02.03; and Delegation of Authority Log - Artifact 02.01.06.

The generation of placeholders based on analysis of documents, such as the Study Protocol, may lead to the analysis of other documents, e.g., documents corresponding to the generated placeholders. For example, if analysis of the Study Protocol produces a placeholder for FDA Form 1572, then analysis of that form (see second row of table of Fig. 2) may result in the generation of further placeholders, such as: 01.01.01 CV for Principal Investigator; 01.01.02 GCP training certs; 02.01.02 Sub-investigator list; 02.01.01 Site Contact List; 05.02.04 Financial Disclosure form; 05.02.01 IRB Approval; 05.02.02 IRB Correspondence; 02.01.06 Delegation of Authority Log; 06.02.01Site Initiation Reports, 02.01.07; Training Logs; 05.01.01 Clinical Trial Agreements; and 05.03.02 FDA correspondence.

The goal of the system is to achieve a high level of automation, with the eTMF being largely autonomous. Various sources of information, such as the initial sales order from a customer (i.e., a user), can trigger the eTMF creation process. Each document within the eTMF, such as the study protocol, contributes specific information for the trial's documentation. The system's design ensures that all necessary documents are automatically generated and organized, significantly reducing the manual workload and enhancing the efficiency of clinical trial management.

In embodiments, the system 100 uses signals and artifacts in conjunction with machine learning models/algorithms, e.g., large language models (LLMs), to generate the structure of the eTMF. Various and multiple types of machine learning models may be utilized within the system, but the typical implementation uses an LLM, which is integrated with the Event Processor 130. This integration seeks to identify signals that inform the creation and organization of artifacts within the eTMF.

For example, signals such as "one study," "five sites," "U.S. only," or "class one medical diagnostic device" are extracted from various inputs. These signals are a basis for determining the specific requirements of the eTMF. Each signal represents a piece of information that contributes to the overall structure and content of the eTMF. The system uses these signals to guide the generation of artifacts, which are the individual documents and data points that populate the eTMF.

### Study Management Application

Another application in the application layer 105 is the Study Management application 155, also known as the Clinical Trial Management System (CTMS). This application is useful for overseeing the entire clinical study. Within the Study Management application 155, various aspects of the study are managed, including, e.g., the selection of study sites, the visibility of these sites, and the definition of study milestones. These milestones are particularly important for the dynamic eTMF system 110, as they determine the timeline for when various documents are due. Since clinical studies often span several years, not all documents are required at the outset. As milestones are reached, the Study Management application 155 sends events to the Event Processor 130, which then updates the eTMF with the necessary placeholders and documents that need to be prepared and uploaded.

During the execution phase of the study, the Study Management application 155 also handles site monitoring. Pharmaceutical companies and life sciences organizations engage with the study sites to ensure adherence to the study protocol. This involves regular site visits to verify compliance, during which documentation is generated. Each site visit is documented in the eTMF, including details such as the date of the visit, the personnel involved, and the findings from the visit. These site monitoring activities produce multiple documents, which are incorporated into the eTMF by sending events to the Event Processor 130. In embodiments, there may be a separate Site Monitoring application 165.

The Study Management application 155 is responsible for generating a significant portion of the many thousands of documents which are added to a typical eTMF over the course of a clinical study. This extensive documentation is important for maintaining regulatory compliance and ensuring the integrity of the clinical trial process. By automating the generation and management of these documents, the system 100 significantly reduces manual workload and enhances the efficiency and accuracy of trial documentation.

### Form Generation

In embodiments, the system 100 may include a form generation module 170 to provide for automatic generation of forms to be stored in the eTMF based on information from other sources, such as an Electronic Data Capture (EDC) system. This capability allows the system to generate necessary forms automatically, further reducing manual workload and ensuring accuracy and consistency in documentation.

For instance, within a clinical study, there are various patient forms that need to be created and filled out. These forms are designed to collect specific data from patients during their visits, such as blood pressure, weight, height, and other relevant health metrics. The study design outlines the required data points, and the resulting predefined forms are integral to the clinical trial process. When a patient arrives for a visit, the system uses the predefined forms to ensure that all necessary data is collected. These forms are tailored to the specific requirements of the study, ensuring that all relevant information is captured accurately.

In embodiments, the system 100 can automatically generate these patient forms, in accordance with the predefined form design, based on data received from other sources. By automating the creation of these forms, the system helps to ensure that important data points are not missed and that the forms are consistently formatted according to the study's specifications. This automation not only enhances the efficiency of data collection but also improves the overall quality and reliability of the clinical trial documentation. It should be noted that in addition to forms containing patient data, it is often the case that blank forms are submitted to be stored in the eTMF to document their structure and edit checks.

### Event Processor/Event Handling

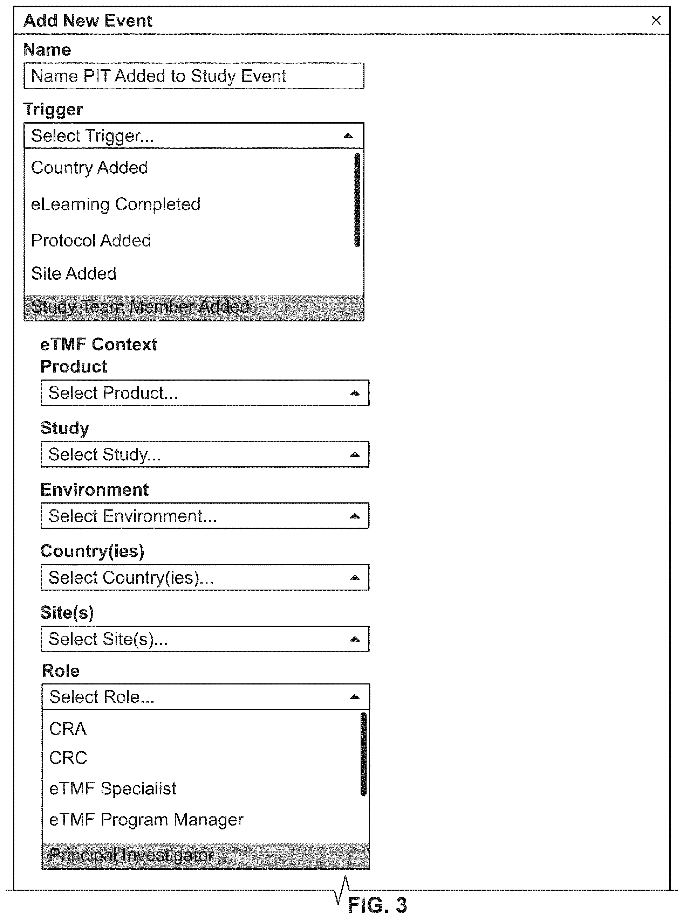

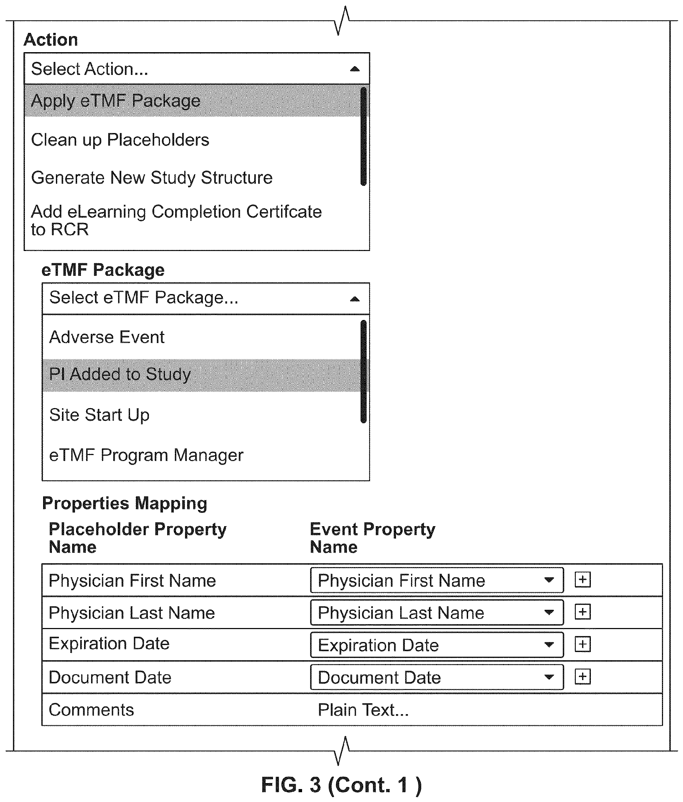

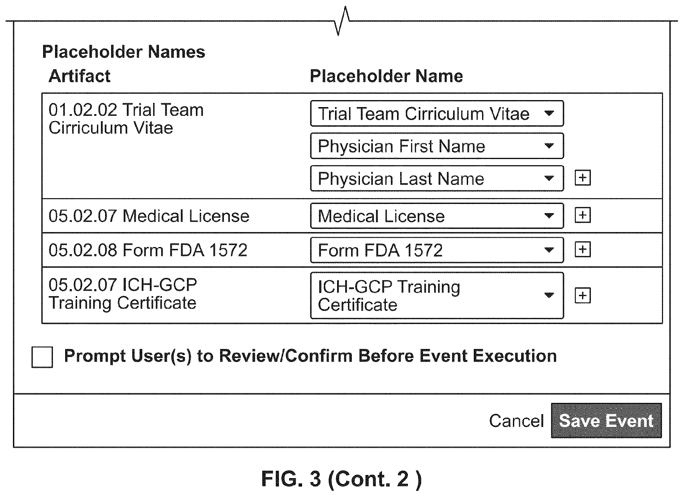

Figure 3 depicts an event handling definition screen to allow input of parameters to be used in event processing. As shown in this example of a user interface, the user can configure event handling for a particular incoming event, which, in this case, is an event that occurs when the Masterdata Management application 125 sends a notification that a study team member has been added. The user can define an event handling to be used by the Event Processor 130 when it receives a notification for this particular event.

To define an event handling, the user enters a name for the event, e.g., "new PIT added to study event" and a trigger, e.g., selected from a pull-down menu which includes: "country added," "e-learning completed," "protocol added," "site added," and "study team member added." In addition, the user enters an "eTMF context" to specify for which product (e.g., a drug), studies, countries, and/or sites this handling would apply. For example, the desired event processing for a particular event may be different in one kind of study, e.g., a study for a pediatric drug, versus another type of study, e.g., a study for a drug which targets adults. As a further example, particular sites in particular countries may have different handling requirements for the same event, e.g., they may require a different set of documents when a study team member is added.

The user specifies a "role," which pertains to event information, where each event can supply additional information. In this instance, we are specifying our interest in cases where a study team member is designated as the Principal Investigator. In embodiments, each event pre-registers meta-information with the system about the event and the additional information it will provide. This user interface of this example displays the specific information registered for the "Study Team Member Added" event.

The user specifies an action to be taken for the specified event and role. In this scenario, we aim to "apply a package," which refers to a collection of placeholders. Based on the selected action, the user can specify the "eTMF package, which is the name of an available package.

The "properties mapping" portion of the user interface allows control over how various properties or metadata associated with the event (which are pre-registered) are mapped to the properties or metadata available for the placeholders in the package. For example, the "Physician's First Name" maps directly, but this would not be the case if the event used "Study Team Member's First Name." The "place holder names" portion of the user interface refers to the placeholders derived from the package and how they will be named. In this case, the value of "Physician First Name" (e.g., "James") from the incoming event (e.g., "James Johnson") will be populated into the "Physician First Name" property (i.e., metadata) of the placeholder. Subsequently, the value will be used as part of the placeholder's name (e.g., "Trial Team Curriculum Vitae James Johnson")

In embodiments, within the eTMF, there are specific artifacts or documents that include lists of other artifacts or documents available in the eTMF. For instance, a "FileNote" is an artifact or document that exists for every zone within the eTMF to document decisions or clarify information related to that zone. Additionally, there is a "FileNote Master List Artifact (01.01.05)." In this case, the generation of this artifact can be automated whenever a FileNote is added to a given zone. This automation occurs when an ad-hoc document, as depicted in Fig. 1, is received and undergoes "document analysis." This analysis creates an event, "FileNote Added," which can have a handler to update the FileNote Master File List. Thus, the addition of a document or placeholder can trigger other processes within the eTMF.

The connections depicted in Fig. 1 may represent a bidirectional flow, in at least some cases, between the dynamic eTMF system 110 and the application layer 105. This is because documents can enter the eTMF containing information that other applications may require. For example, the Monitoring Plan (Artifact 01.01.08) contains information that is needed by the Clinical Trial Management System (CTMS). In this instance, the dynamic eTMF system 110 can send out an event indicating that the Monitoring Plan is available, allowing the CTMS to respond by automatically configuring the monitoring system accordingly.

The Monitoring Plan is a document that outlines how monitoring will be implemented during a clinical trial, including strategies for source data verification. This artifact may include any evidence of plan execution, such as the plan itself, reports, checklists, and other related documents. Typically, the Monitoring Plan is one of the plans managed by Clinical Operations (clinops). However, the document is not usually stored within CTMS. Rather, it is used as a reference when setting up parameters such as monitoring visit frequency and visit types. The Monitoring Plan is typically created before the study can be executed in CTMS, and, outside of the eTMF, there may be no convenient repository for this document. In some cases, there may be a link provided in CTMS to facilitate easy access to the document within the eTMF.

Because the CTMS is configured based on the content of the Monitoring Plan, and Clinical Research Associates (CRAs) frequently reference it, it may not be desirable to recreate the document based on the settings in CTMS. Therefore, in implementations, users may leverage document tracking combined with edit capabilities to create the Monitoring Plan. Thus, as discussed above, it may be more effective for the system to configure the study in CTMS based on the document stored in the eTMF, according to disclosed embodiments.

### Use of TMF Reference Model

As discussed above, the typical implementation uses an LLM, which is integrated with the Event Processor 130. This integration seeks to identify signals that inform the creation and organization of artifacts within the eTMF. Part of the process involves mapping signals to specific artifacts. This mapping can be straightforward in some cases, where a direct correlation exists between a signal and an artifact. However, in other cases, the mapping may be more complex and require machine learning/artificial intelligence techniques to interpret and apply the signals accurately. The use of LLMs allows the system to process these signals and generate the necessary artifacts, ensuring that the eTMF is comprehensive and correctly structured.

To facilitate this process, the system 100 may rely on a detailed TMF Reference Model, which includes all of the available artifacts and the corresponding signals needed to generate them. For example, the system 100 may use the Reference Model maintained by the Clinical Data Interchange Standards Consortium (CDISC), which is the industry standard. This structured approach ensures that the system can accurately and efficiently create the eTMF based on the provided data.

### Typical Considerations in Generating the eTMF

As discussed above, conventional eTMF systems manually create the eTMF structure with placeholders after customizations that can take weeks to carry out. In contrast, the embodiments described herein allows for the automatic configuration of a functional eTMF based on various input parameters, which will gradually populate the structure.

For instance, the system takes into account the Trial Phase. Early-phase trials (Phase I) may not require as much documentation as later phase trials (Phase III), especially in terms of safety reporting and regulatory approvals. This differentiation ensures that the eTMF is tailored to the specific requirements of each trial phase, streamlining the documentation process.

The Type of Trial is another key parameter. Interventional trials, such as those testing new drugs, generally require a more comprehensive set of documents compared to observational studies, which might involve less direct involvement with participants and lower regulatory scrutiny. For non-drug trials, such as medical device trials or behavioral interventions, certain documents like the Investigator's Brochure may not be applicable. By recognizing these distinctions, the system ensures that only relevant documents are included in the eTMF, enhancing efficiency.

Regulatory requirements vary across regions, such as the FDA in the U.S., EMA in the EU, and PMDA in Japan. The specific documents needed will depend on the jurisdiction, and some studies, like non-interventional or post-marketing surveillance, may not require as stringent documentation. The system automatically adapts to these regional differences, ensuring compliance with local regulations without the need for manual customization.

The Risk Level of the trial also influences the documentation requirements. Low-risk trials, such as those studying already approved treatments in a new population, may have fewer documentation requirements. Conversely, high-risk trials involving novel drugs or gene therapies demand more comprehensive documentation, including additional safety reports. By adjusting the eTMF structure based on risk level, the system ensures that all necessary documentation is included without overburdening low-risk trials with unnecessary paperwork.

In summary, the autonomous eTMF generation and modification system described herein provides a significant advancement over traditional methods by automating the creation and population of the eTMF structure. By considering parameters such as trial phase, type of trial, regulatory requirements, and risk level, the system ensures that the eTMF is tailored to the specific needs of each clinical trial. This automated, dynamic approach enhances efficiency, accuracy, and compliance, offering a robust technical solution to the challenges of managing clinical trial documentation.

### Real-time Event-Driven Generation of eTMF Structure.

In embodiments, the user will initially see a minimal eTMF structure being created when the study is being defined. Subsequently, placeholders and content will be added based on various events in the other applications integrating with the eTMF. This dynamic and event-driven approach helps ensure that the eTMF evolves in real time, reflecting the latest developments and requirements of the clinical trial.

### Processing of Events by Event Processor

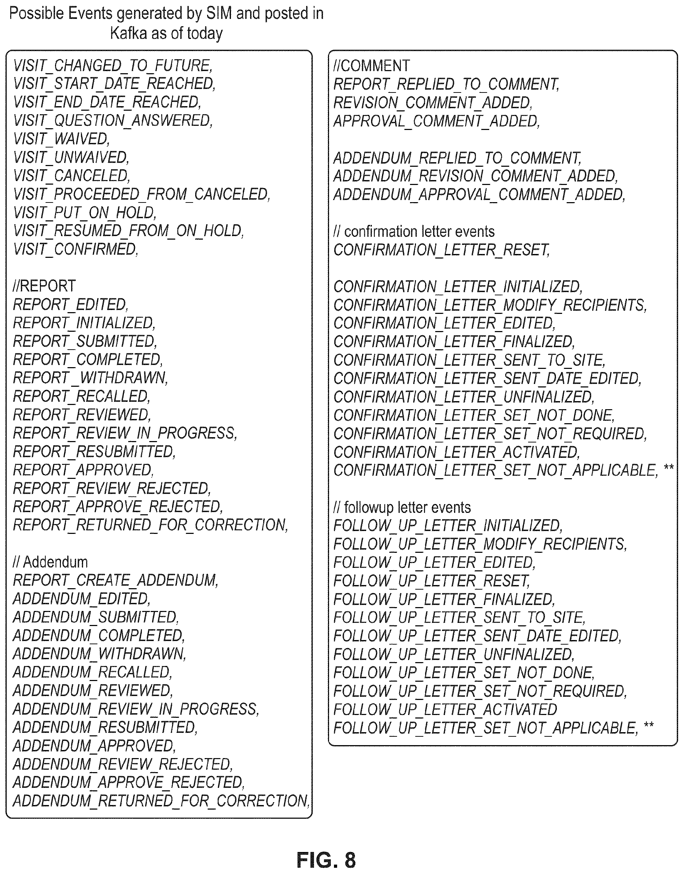

Referring again to Fig. 1, the Event Processor 130 is configured to process a set of events generated by the Study Management Application 155, such as those listed in Fig. 8. In embodiments, these events may include milestones such as "REPORT_ INITIALIZED," "VISIT_CONFIRMED," and "CONFIRMATION_LETTER_INITIALIZED," among others. These events are posted by the message system 132 (e.g., Kafka) and processed by the Event Processor 130 to dynamically create placeholders, manage document workflows, and update trial progress in real time. For example, when a "VISIT_CONFIRMED" event occurs, placeholders for confirmation letters and related visit documentation are generated automatically in the eTMF file structure 112. This streamlined processing helps ensure all relevant artifacts are prepared and managed according to pre-established protocols and regulatory requirements. By leveraging the predefined event types in the Study Management Application 155, e.g., as shown in Fig. 8, the system supports comprehensive coverage of trial activities and documentation needs, minimizing delays caused by manual operations and helping to ensure operational readiness throughout the trial lifecycle.

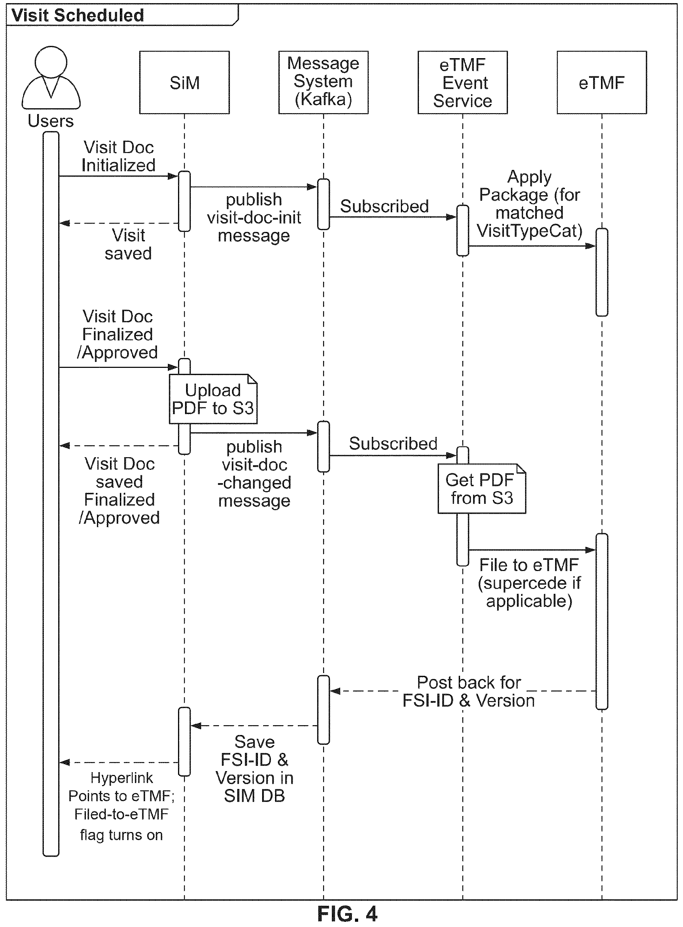

The interaction between various system components, as depicted in Fig. 4, highlights how the event-driven architecture accelerates milestone-driven workflows for placeholder generation and document management. For example, when a user initializes a visit document through the Study Management Application 155, a "Visit Document Initialized" is published to the message system 132 (e.g., Kafka), as shown in the first sequence of Fig. 4. This event triggers the Event Processor 130 to apply the appropriate package corresponding to the visit type category, dynamically creating the necessary placeholders within the eTMF file structure 112. Additionally, when the user finalizes or approves the visit document, e.g., via the "finalize" or "approve" operations in CTMS, as illustrated in the second sequence of Fig. 4, the finalized document is uploaded to a cloud service (e.g., S3) and a "Visit Document Changed" message is published to the message system 132. The Event Processor 130 retrieves the finalized document from S3, files it into the dynamic eTMF system 110 (using the associated placeholder), and supersedes prior documents if applicable. This interaction helps ensure real-time updates to eTMF placeholders while maintaining audit and versioning capabilities.

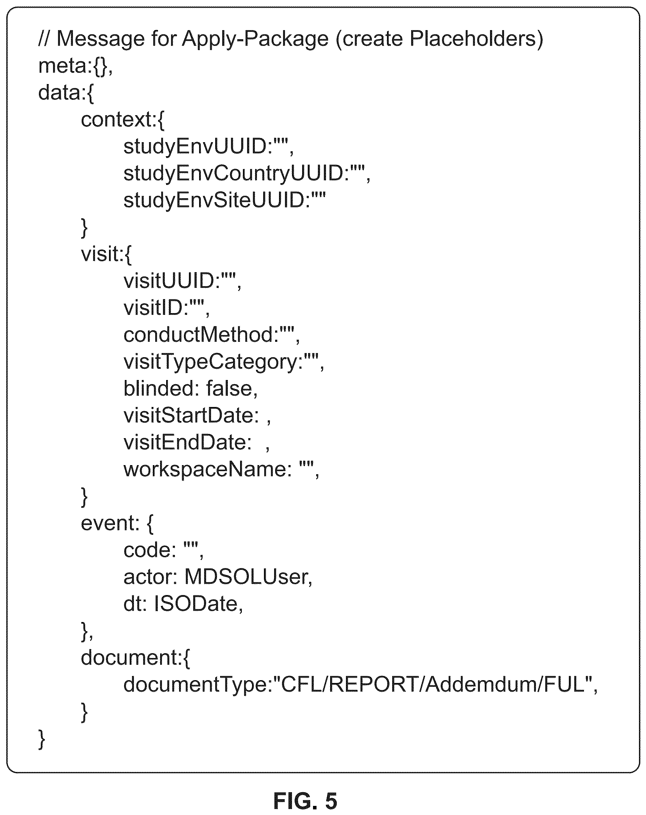

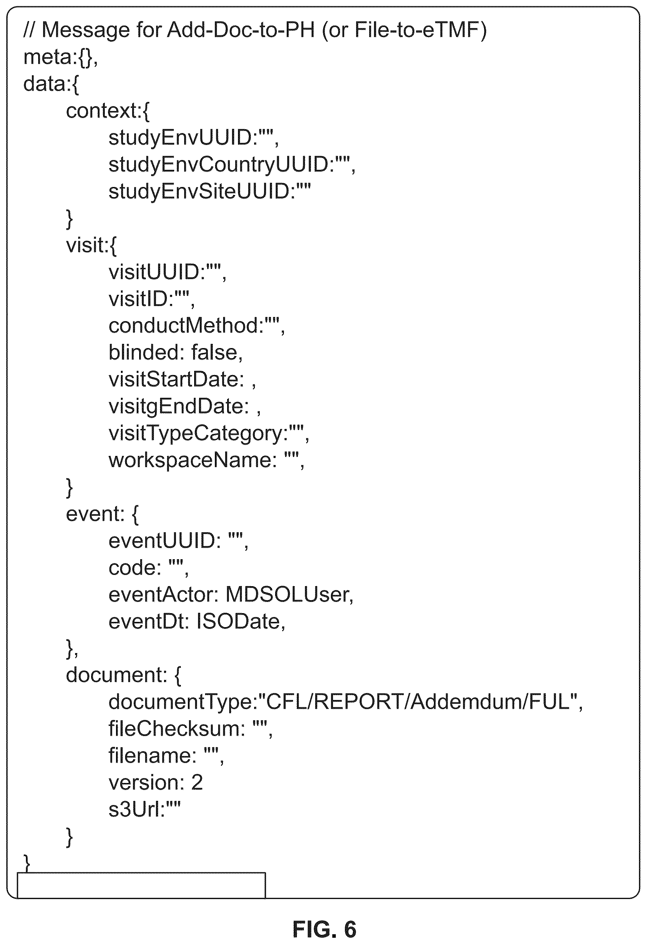

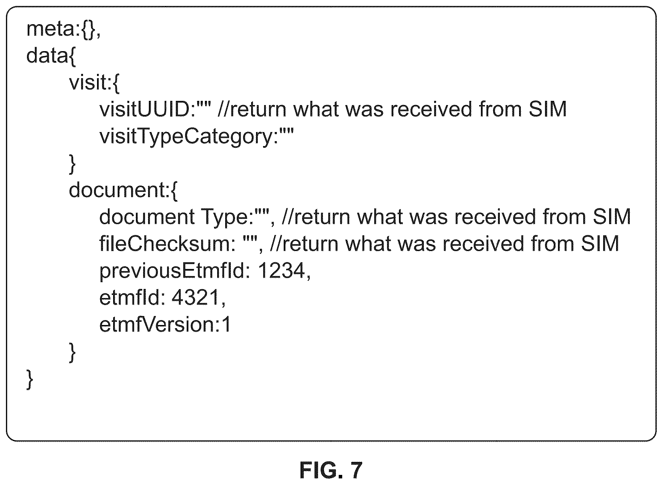

Figs. 5-7 are schemas for use by the Event Processor 130 to perform event handling via the message system 132. The Event Processor 130 facilitates advanced integration across milestones, placeholders, and document fulfillment workflows using schemas, such as the "apply-package" message (Fig. 5) and "add-doc-to-PH" message (Fig. 6). For example, when a "REPORT_SUBMITTED" event occurs for a visit report, the Event Processor 130 ensures that the corresponding placeholders in the eTMF file structure are dynamically updated, and the document is positioned accurately. In embodiments, a document confirmation schema, e.g., the "return what was received" schema of Fig. 7, may be used to confirm that the document was successfully stored and associated with the correct placeholder. This robust event-driven mechanism ensures real-time updates across large, distributed trials, improving data integrity and operational efficiency. By managing dependencies between events such as "VISIT_START_DATE_REACHED" and "CONFIRMATION_LETTER_INITIALIZED" (see Fig. 8), the system enables nuanced placeholder creation that reflects actual trial progress, helping to ensure that documentation workflows remain precise and audit-ready, even in trials with complex timelines and diverse document needs.

In embodiments, the use of dynamic event dependencies allows the system to handle phased document workflows more effectively. For example, a "VISIT_ CONFIRMED" event triggers the generation of placeholders for visit scheduling and initial documentation, while subsequent events like "VISIT_START_DATE_REACHED" dynamically add placeholders for safety reports, monitoring logs, and site-specific regulatory submissions. By managing these interlinked dependencies across multiple trial phases, the system ensures that placeholders are created as needed rather than upfront, reducing document clutter and providing focused, phase-specific compliance tracking. This phased approach aligns with operational needs and ensures that documentation evolves in tandem with trial progression.

The event-driven architecture facilitates collaborative eTMF management across multiple trial stakeholders. For instance, when a document placeholder is fulfilled (e.g., through a "file-to-eTMF" message), the system updates other integrated systems such as the Study Management Application 155 or Adverse Events Handler 135 in real time. These updates ensure synchronization across systems and provide stakeholders with access to the most current information. Additionally, events such as "VISIT_START_DATE_REACHED" or "CONFIRMATION_LETTER_FINALIZED" trigger automatic notifications and updates to associated teams, facilitating seamless collaboration and reducing latency in document workflows. This integrated, event-driven approach enhances transparency and coordination among trial personnel, regulatory teams, and external partners, helping to ensure eTMF completeness and real-time alignment with trial milestones.

In embodiments, the system leverages machine learning techniques to enhance event processing and document management workflows. For example, the Event Processor 130 may use machine learning algorithms to analyze historical and real-time data from events (see, e.g., Fig. 8) to predict potential delays in placeholder creation or document submission. If a specific event type, such as "VISIT_CONFIRMED," has historically delayed the creation of confirmation letter placeholders, the system preemptively allocates resources to address this gap. Additionally, machine learning integration ensures that placeholders requiring document localization (e.g., language-specific versions for global sites) are dynamically flagged and adapted in future workflows. The use of machine learning facilitates continuous learning and improvement in system workflows, enabling adaptive automation that evolves alongside trial conditions and regulatory expectations.

### Creation of File Plan and Placeholders

Fig. 5 provides an example of a schema for an "apply-package" message used to create eTMF placeholders on detecting relevant events in the Study Management Application 155. For example, this message may include attributes such as placeholder categories, study context, event type, and application source, helping to ensure alignment between the placeholders created and the triggered clinical trial milestone. Once the message is processed by the Event Processor 130, hierarchical placeholder structures are dynamically established within the eTMF file structure 112. When finalized or updated documents need to be filed into these placeholders, the Event Processor uses an "add-doc-to-PH" (or "file-to-eTMF") message (see Fig. 6) to ensure accurate document placement. This approach reduces human error and supports version control by superseding outdated files where applicable.

In embodiments, the system optimizes file plan creation and placeholder generation by tailoring the eTMF structure to align with trial phases, as shown through workflows in Fig. 4. For early-phase trials (e.g., Phase I), the system may limit placeholders to basic safety and protocol documentation, while in later phases (e.g., Phase III) the system may generate additional placeholders for complex regulatory filings, expanded site reports, and final study results. This phase-specific approach ensures that the eTMF evolves dynamically along with the trial's progression, minimizing placeholder clutter at earlier stages while addressing the increasing demands of later trial phases.

To accommodate broader trial requirements, the system includes an extensive library of event types that can trigger placeholder generation and document updates. Fig. 8 is a list of examples of message system events (or "topics") which are used by the Event Processor 130 in event handling. This includes events such as "REPORT_INITIALIZED," "VISIT_START_DATE_REACHED," and "CONFIRMATION_ LETTER_INITIALIZED." The events enable granular tracking of trial progress, with each event pre-configured to generate specific placeholders via the Event Processor 130. By referencing schemas like "apply-package" (Fig. 5) and "add-doc-to-PH" (Fig. 6), the system provides sponsors with a customizable approach to document management based on unique trial workflows. These updates ensure timely fulfillment of documentation requirements while supporting multi-layered tracking and compliance oversight during trial execution.

In embodiments, placeholder creation is progressively aligned with trial milestones using messaging frameworks, such as those depicted in Figs. 4-8. For example, during the startup phase of the trial, placeholders are generated for site initiation documents and principal investigator credentials based on initial events such as "SITE_ADDED" or "PI_ASSIGNED." Later in the course of a trial, as milestones such as "PATIENT_ENROLLED" or "SITE_CLOSEOUT" are reached, additional placeholders such as patient safety reports, closeout visit reports, or final monitoring logs are progressively created. This progressive approach ensures that placeholders remain relevant to the current trial phase, reducing the complexity of managing unnecessary placeholders early on. By dynamically adjusting the eTMF structure in response to milestones, the system minimizes document clutter and enhances organizational efficiency throughout lengthy trial timelines.

In embodiments, timing-based processes are integrated into the system to ensure placeholders are created and fulfilled in alignment with the trial's critical risk points. As depicted in Fig. 4, messaging workflows ensure placeholders tied to high-risk milestones, such as "PATIENT_SCREENED" or "VISIT_REPORT_FINALIZED," are dynamically generated as events occur. The timing mechanism prevents premature placeholder creation (which could flood the eTMF with unnecessary placeholders early on) while helping to ensure that applicable documentation is declared as expected at the right time during the clinical trial. This approach mitigates common issues in document management for trials, such as presenting a false-sense of incompleteness by prematurely expecting too many documents across long-running studies.

In embodiments, the Event Processor adapts placeholder generation and document workflows dynamically based on phase transitions. For example, when transitioning from Phase I to Phase II, milestones such as "PHASE_I_COMPLETE" may trigger automatic updates to the eTMF structure, including new placeholders for expanded regulatory submissions, interim safety analyses, and updated protocols. These adaptive triggers ensure that placeholders are created and documents are managed only when necessary, reducing clutter while aligning eTMF operations with the evolving requirements of the trial phase. This functionality reduces manual adjustments during phase transitions, ensures regulatory compliance, and maintains operational efficiency for trials spanning multiple phases.

In embodiments, the system employs role-based dynamic document management, leveraging particular events, such as those shown in Fig. 4. For instance, when roles such as "Study Coordinator" or "Principal Investigator" are added for a particular site, the Event Processor 130 dynamically generates placeholders tied to each role's responsibilities and sends task-specific notifications to the assigned personnel. By automatically delegating document management tasks based on role, the system reduces administrative overhead and ensures that trial personnel focus on relevant, actionable items for their specific site and role.

In embodiments, the system facilitates seamless interaction with external document repositories to fulfill placeholders efficiently. For example, when triggered by events like "TRAINING_ASSIGNED," the Event Processor will create matching placeholders for assigned personnel. Upon a subsequent "TRAINING_COMPLETED" event, the corresponding training evidence (e.g., a Training Course Certificate) may be downloaded from an external source (e.g., a third-party training program) via secure APIs.. The system then files these documents in the appropriate previously-created placeholders within the dynamic eTMF system 110. This integration significantly reduces manual effort in document retrieval and improves synchronization with external collaborators, making it easier to maintain a complete and compliant eTMF.

### Document Processing

In embodiments, a document validation layer ensures adherence to pre-defined standards following placeholder fulfillment. For example, when a document is uploaded via the workflows, such as those depicted in Fig. 4, the system cross-references document format, content, and consistency with the associated placeholder metadata. Validation errors, such as missing signatures or incomplete attachments, may be flagged, and notifications may be sent to the relevant personnel through the Study Management Application 155. This validation process helps to ensure that placeholders are not marked as complete unless the uploaded documents meet regulatory and operational criteria. By integrating document validation directly into the placeholder fulfillment workflow, the system helps to prevent incomplete or non-compliant documents from disrupting trial timelines or jeopardizing regulatory readiness. This validation layer, combined with the event-driven workflows, establishes a robust mechanism for ensuring the quality and accuracy of submitted documents while maintaining traceability throughout the eTMF lifecycle.

The system may employ intelligent document categorization using machine learning models and event-driven signals. For example, when a placeholder is fulfilled via a "file-to-eTMF" message (see Fig. 6), the system analyzes document metadata, such as type, author, and associated milestone, and categorizes it into the appropriate directories within the eTMF structure 112.

The bidirectional message flow depicted in Fig. 4 illustrates how the system provides a continuous audit-ready feedback loop from placeholder creation to document filing. For instance, as shown in the second sequence of Fig. 4, once a document is successfully stored in the eTMF system, the system generates a "post back" message that is routed through the message system 132 to confirm the document identifier (FSI-ID) and version of the document. This message flow helps to ensure that the Study Management Application 155 database reflects the most recent document status. Real-time confirmation of document filing status allows for seamless coordination between site personnel, regulatory teams, and trial sponsors, helping to ensure that placeholders are fulfilled timely for high-priority trial milestones.

The sequence diagrams and schemas described herein illustrate the ability of the system to provide integrated feedback on study documents and placeholder status. For instance, as milestones trigger document uploads (e.g., "REPORT_SUBMITTED" or "VISIT_START_DATE_REACHED"), the feedback loop ensures that placeholders are accurately updated and flagged as fulfilled, reducing inefficiencies in document tracking and submission. The Event Processor 130's ability to interactively retrieve, validate, and store documents allows trial sponsors to maintain continuous compliance visibility, while post-back mechanisms ensure end-to-end consistency between the message system 132, Study Management Application 155, and the dynamic eTMF system 110.

The event workflows described herein enhance collaboration across integrated applications by facilitating seamless communication of placeholder status and document updates. When events like "TRAINING_ASSIGNED" or "TRAINING_COMPLETED" occur in the Study Management Application 155, the Event Processor dynamically updates the eTMF, while also synchronizing with relevant applications such as the Adverse Events Handler 135. Furthermore, the granular reporting built into the message system 132, as described, e.g., in Figs. 4-8, enables stakeholders to track document states with high precision. For example, when a visit report is updated, the system generates real-time status updates in the form of "return what was received" messages (see Fig. 7) which include details about the document's current state, placeholder assignment, and version history. This feedback loop helps ensure that document updates are reflected across all integrated applications, including the Study Management Application 155 and user interfaces. This enhanced reporting functionality provides trial sponsors and site managers with clear visibility into document workflows, improving overall operational transparency and reducing risks of incomplete or outdated content.

In embodiments, document versioning capabilities track updates in real time. For example, when a placeholder for a visit report is fulfilled with a new version of the document, the system supersedes the older version and may archive it for compliance purposes. Post-back confirmation messages, e.g., according to the schema of Fig. 7, provide users with traceable evidence of the versioning process, helping to ensure that regulatory bodies have access to an accurate document history during audits. This expanded version control system improves oversight, ensures alignment with regulatory standards, and reduces risks of errors related to outdated or duplicate documents.

Document versioning and linking workflows, e.g., as described in the second sequence of Fig. 4, enable the system to maintain real-time alignment between placeholders and uploaded documents. For example, after the Event Processor retrieves and files a finalized document into the eTMF system, the system automatically validates the document version and generates a "Post Back for FSI-ID & Version" message, as shown in Fig. 4. This integration prevents duplication errors and ensures that trial personnel can access the latest version of filed documents directly through hyperlinks generated by the Study Management Application 155 (as noted under "Hyperlink Points to eTMF"). These advancements solidify the system's ability to maintain comprehensive, version-controlled documentation that is accessible, accurate, and aligned with regulatory requirements.

In embodiments, the system integrates a detailed audit trail for version control and event tracking based on workflows, e.g., as described in Fig. 4. Each document uploaded, updated, or superseded may be logged with a timestamp, associated milestone, and event metadata. By maintaining a complete history of placeholder interactions-such as when they were created, fulfilled, and linked to finalized documents-the system ensures regulatory bodies have traceable evidence of compliance during inspections. This audit trail, paired with dynamic post-back verification mechanisms, provides trial sponsors with complete transparency into trial operations, facilitating compliance assurance while enabling rapid response to external audits or internal inquiries.

### Risk-based and Predictive Event Handling

The system supports multi-layered event handling for risk-based milestones, leveraging particular event types (see, e.g., Fig. 8) and their integration with placeholder workflows. For example, when a high-risk event such as "ADVERSE_EVENT_REPORTED" is triggered, the system creates placeholders for safety documentation at both the study and site levels. Parallel workflows ensure that required placeholders are created simultaneously across all sites, helping to ensure that critical events affecting patient safety and regulatory submissions are prioritized. This multi-layer integration aligns milestone scheduling with compliance-critical tasks, enhancing system reliability and preparedness for complex trial operations.

### Localized Policy Enforcement and Workflow Customization

The system equips the Event Processor 130 with localized policy enforcement capabilities aligned with region-specific milestone requirements. For example, placeholders generated for clinical trials in Germany may include specific metadata and document requirements tailored to national and/or regional laws and regulations, enforced automatically during placeholder creation (see, e.g., Fig. 5) and document filing workflows (see, e.g., Fig. 6). Similarly, for sites in the United States, placeholders align with FDA-specific regulatory standards, helping to ensure that compliance tasks are tailored to the applicable jurisdiction. By dynamically aligning placeholder packages with geographic regulations, the system ensures global compliance while avoiding redundant or irrelevant documentation. This precision reduces manual configurations and strengthens the eTMF's adaptability to multi-region trials, improving setup efficiency and minimizing compliance errors.

Using the workflows, schemas, and message system topics, as depicted, e.g., in Figs. 4-8, embodiments of the system dynamically support region-specific workflow customization based on localized regulatory requirements. For example, events like "SITE_ADDED" or "PI_ASSIGNED" trigger placeholder creation tailored to the applicable jurisdiction's standards. This regional tailoring ensures regulatory compliance globally, simplifies audits for international trials, and reduces manual adjustments required by trial coordinators to address diverse regulatory landscapes.

### Advanced Site Monitoring, Metrics, and Notifications

The system supports a global fulfillment view for sponsors to track placeholder progression across all participating sites. Leveraging event messaging workflows and fulfillment confirmation messages from (see, e.g., Fig. 4), sponsors can access a centralized dashboard detailing fulfilled, pending, or overdue placeholders for each site. For example, sponsors can review performance at a glance, identifying sites with outstanding critical documents like ethics committee approvals or safety reports. The dashboard aggregates data into meaningful insights and highlights areas requiring intervention, enabling sponsors to prioritize resources effectively and ensure trial milestones are not delayed.

In embodiments, a real-time escalation framework triggered by particular events (see, e.g., Fig. 8) alerts sponsors and regulatory teams when critical placeholders risk breaching compliance deadlines. For example, if placeholders tied to "REGULATORY_ SUBMISSION" remain unfulfilled within a specified timeframe, the system escalates the issue to higher-level personnel, such as trial managers or compliance officers. These escalations include contextual metadata from events such as "MILESTONE_DUE_DATE_REACHED," enabling the recipient to act with important background information. The framework minimizes oversight delays in high-priority areas and strengthens overall compliance management for sensitive trial phases.

Advanced site monitoring metrics, integrated with workflows (see, e.g., Fig. 4), may be used to provide granular feedback on site-specific document status and long-term performance. For example, for each site, the system may track metrics such as placeholder fulfillment rates, document submission delays, and frequency of report revisions. Events such as "VISIT_CONFIRMED" or "REPORT_SUBMITTED" generate real-time updates to these site-specific metrics, enabling trial managers to identify problematic sites and address performance issues proactively. These granular insights allow sponsors to assess trial health at the site level, optimize resource allocation, and improve overall compliance and operational efficiency across all sites involved in the trial.

In embodiments, the system features automated milestone-specific notifications designed to alert trial personnel about tasks tied to upcoming or overdue milestones. For example, when the "PATIENT_SCREENED" event is triggered, the system sends targeted notifications to relevant site administrators, reminding them of placeholders for patient consent forms and screening safety reports. These notifications, generated via the Event Processor and routed using workflows, may be role-specific and may include contextual data such as document deadlines and procedural requirements. By proactively notifying personnel of milestone-specific document expectations, the system ensures timely placeholder fulfillment, reducing delays and supporting seamless regulatory compliance.

The system provides notification and monitoring features which allow sponsors to compare placeholder fulfillment metrics between trial sites. For example, the system can highlight sites with faster turnaround times for document submissions, such as visit reports or training certifications, while flagging underperforming sites for intervention. This cross-site comparison feature offers actionable insights, enabling sponsors to identify best practices at high-performing sites and apply them to improve lagging sites. It also aids in dynamic resource allocation, helping to ensure that critical trial activities stay on schedule regardless of geographic scope.

Using query-driven workflows, supported by the messaging schemas discussed above, the system allows sponsors to generate on-demand compliance reports. These reports pull real-time data on placeholder status, document versioning, milestone alignment, and event-driven updates across all integrated components. For example, during a regulatory audit, sponsors can query placeholders tied to specific milestones like "SITE_SELECTED" or "SITE_CLOSED_OUT" and retrieve the corresponding filing history. This capability ensures that sponsors remain audit-ready, while eliminating manual searches and delays during regulatory inspections.

In embodiments, the system integrates automated risk profiling for site compliance, leveraging event-driven workflows and metrics tracking. For example, the Event Processor analyzes past event data, such as delays in "VISIT_CONFIRMED" or "REPORT_SUBMITTED" events, to score sites based on their historical performance and timeliness of fulfilling placeholders. Low-compliance sites are flagged for additional monitoring or intervention during future milestones, such as document submissions or regulatory approvals. This automated risk profiling helps sponsors proactively address potential issues with underperforming sites, improving overall compliance and helping to ensure a smooth workflow across all trial locations.

Using combined data tracked through placeholders and document submission patterns, the system may offer performance insights into the activities of external vendors and regulatory interactions, as managed via the message system 132. For example, the system may track metrics for vendor-submitted documents, such as lab test results or ethics board reviews, to evaluate the timeliness and completeness of submissions. Sponsors can use these insights to identify high-performing vendors or address delays from underperforming collaborators. This transparency improves coordination across stakeholders and ensures that regulatory submission deadlines are consistently met at all sites.

Integrated messaging flows, as described above, allow the system to track placeholders and document fulfillment from initialization to completion. For instance, when a placeholder corresponding to a milestone (e.g., "VISIT_CONFIRMED") is dynamically created through an "apply-package" message (see, e.g., Fig. 5), the status of the placeholder is updated in real time through subsequent messages sent by the Event Processor 130, such as a "file-to-eTMF" message (see, e.g., Fig. 6). As fulfilled placeholders are linked to uploaded documents, discrepancies can be flagged to help ensure that all necessary artifacts required for compliance are addressed. By monitoring placeholder states via bidirectional messaging flows, the system accounts for dependencies between site-specific activities, establishing greater accountability and efficiency in trial documentation handling across study sites.

In embodiments, the system may include a high-priority notification framework to alert users of critical placeholder activity. For example, when placeholders tied to deadlines, such as "REGULATORY_APPROVAL_REQUIRED" or "ETHICS_ COMMITTEE_APPROVAL_OUTSTANDING," approach their due date, the system triggers notifications to responsible personnel. These notifications include actionable details such as the placeholder, relevant milestone description, and expected document. This high-priority notification system helps ensure the timely submission of compliance-critical documents, reducing the risk of delays that could compromise regulatory timelines.

In embodiments, the system integrates a visual dashboard that displays compliance gaps in real time, leveraging placeholder fulfillment data and event feedback. For example, placeholders flagged as overdue are highlighted on the dashboard, along with their associated milestones and documents. Users can drill down into site-specific details, viewing document submission timelines and fulfillment metrics for critical milestones like "REGULATORY_APPROVAL" or "PATIENT_SCREENED." This visualization tool provides trial sponsors with actionable insights into the eTMF's progress, enabling faster resolution of compliance issues and helping to ensure timely adherence to regulatory requirements.

The event-driven structure of the system is beneficial in scenarios in which a clinical trial must adjust parameters, such as participant enrollment or dosing strategies, based on interim results. For example, when an adaptive trial milestone like "TRIAL_ PROTOCOL_AMENDED" is detected, the Event Processor 130 may dynamically update placeholders to reflect new documentation needs, including an updated trial protocol or additional regulatory submissions. This real-time adaptability helps to ensure that the eTMF evolves efficiently to match the changing requirements of the trial, reducing administrative overhead and helping to ensure ongoing compliance with regulatory standards.

By incorporating predictive algorithms into its event-driven workflows, the Event Processor 130 can anticipate potential bottlenecks in placeholder fulfillment. For example, using data from past events, such as "REPORT_INITIALIZED" and "VISIT_ START_DATE_REACHED," the system identifies patterns that indicate delays in document preparation or submission at specific milestones. Where delays are predicted, the Event Processor 130 may trigger preemptive notifications, suggesting actions to mitigate risks or allocate resources to high-risk workflows. This predictive capability helps to ensure smoother trial progression by addressing potential delays before they occur, enhancing operational efficiency and reducing regulatory risks tied to incomplete trial documentation.

In embodiments, the system may integrate e-Learning progress tracking with the event workflows (see, e.g., Fig. 4). For example, when training milestones such as "TRAINING_COMPLETED" are reached within the e-Learning Management application 160, the Event Processor 130 automatically updates corresponding placeholders in the eTMF for training certifications. As training-related placeholders are fulfilled, the Event Processor may communicate the status back to the e-Learning Management application, helping to ensure that both systems are synchronized. Notifications may be sent to site administrators and sponsors, enabling them to track training compliance in real time. This integration helps to ensure that staff at all sites meet required qualifications before participating in clinical trial activities, thereby reducing regulatory risks and helping to ensure readiness for inspections.

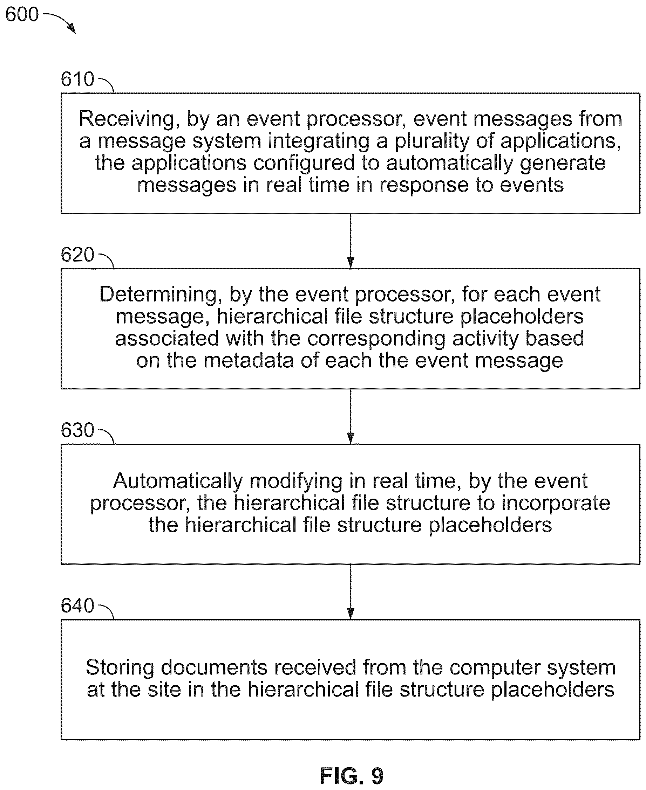

Fig. 9 is a flowchart of a method for autonomous real-time generation and modification of a dynamic electronic Trial Master File, according to disclosed embodiments. The method 600 includes receiving, by an event processor, event messages from a message system integrating a plurality of applications, the applications configured to automatically generate messages in real time in response to events (610). Each of the events corresponds to a user interaction with one of the applications via a user interface of a computer system at a first site based on an activity associated with the clinical trial. Each event message, corresponding to each user interaction, includes metadata relating to a corresponding activity. The method further includes determining, by the event processor, for each event message, one or more hierarchical file structure placeholders associated with the corresponding activity based on the metadata of each said event message (620). The method further includes automatically modifying in real time, by the event processor, the hierarchical file structure to incorporate said one or more hierarchical file structure placeholders (630). The method further includes storing one or more documents received from the computer system at the first clinical trial site in said one or more hierarchical file structure placeholders (640).

### Disclosed Embodiments Provide Technical Solution to Technical Problem

The autonomous electronic Trial Master File (eTMF) system is a technical solution addressing the technical problem of efficiently managing and organizing clinical trial documentation. Traditional eTMF systems are highly manual and labor-intensive, leading to inefficiencies, delays, and increased risk of errors. The disclosed embodiments resolve these issues through an innovative integration of automation, leveraging signals and data to autonomously create and populate the eTMF. This system constitutes a technical solution to a technical problem for several reasons.

The system automates the document management process, which traditionally requires significant manual effort. Manual processes in conventional eTMF systems are prone to human error, inconsistencies, and inefficiencies, contributing to delays and increased costs in clinical trials. The autonomous eTMF generation and modification system mitigates these issues by using signals and data to dynamically create placeholders for required documents and automatically populate these placeholders as data and documents become available. This automation significantly reduces the need for manual intervention, thereby minimizing errors and inconsistencies and ensuring timely updates. This structured automation provides a clear technical advancement over traditional manual systems.

The system integrates multiple applications, such as e-consent, adverse events, and study management, into a unified framework. Clinical trials involve various aspects, each managed by different applications, and integrating data from these disparate sources into a cohesive eTMF is a complex and time-consuming process. The autonomous eTMF generation and modification system processes signals from these applications to automatically generate and organize the necessary documentation, ensuring comprehensive and up-to-date records. This integration allows for seamless data flow and real-time updates across different components of the clinical trial process, representing a substantial technical enhancement in clinical trial management.

The system employs large language models (LLMs) to generate signals and extract information required for the eTMF and other applications. Extracting and processing relevant information from diverse and unstructured data sources is a significant challenge in managing eTMFs, and traditional systems lack the capability to efficiently interpret and utilize this data. LLMs can interpret complex data inputs and map them to specific artifacts needed for the eTMF, allowing the system to autonomously generate documents and organize them based on predefined criteria, ensuring accuracy and completeness. This capability goes beyond generic data processing, providing a sophisticated method for managing clinical trial documentation.

The system uses templates and event-driven processing to dynamically create documents based on contextual data. Clinical trials require the creation of various documents at different stages, such as patient forms, training certificates, and adverse event reports, and manually generating these documents is inefficient and error-prone. For example, when a patient completes training, the system can automatically generate a training certificate using stored data and a predefined template. This approach ensures that documents are created accurately and promptly, reducing delays and manual errors. The use of templates for dynamic document creation is a specific technical feature that improves the reliability and efficiency of the eTMF system.

The system includes a centralized document repository that allows documents to be stored once and reused across multiple studies. Managing documents across multiple studies and ensuring their availability for reuse is challenging in traditional systems, often leading to redundant efforts and increased storage requirements. For instance, a medical license for a principal investigator can be stored in the repository and referenced in any study involving that investigator, reducing redundancy and ensuring that the latest version of the document is always used. This centralized approach optimizes storage and ensures consistency, enhancing the overall management of clinical trial documentation.

Additionally, the system ensures regulatory compliance and audit readiness by maintaining a comprehensive and up-to-date eTMF. Ensuring regulatory compliance and maintaining audit readiness are challenges in clinical trials, and traditional eTMF systems often struggle to provide real-time access to complete and accurate documentation. By automating the document management process and maintaining a comprehensive repository, the autonomous eTMF generation and modification system enhances regulatory compliance and audit readiness, providing real-time access to accurate and complete documentation.

For example, if an adverse event requires new documentation across trial sites, the system ensures that placeholders for these reports are created simultaneously at all relevant locations. This synchronization minimizes delays in multi-application workflows and ensures all trial components operate cohesively in line with regulatory expectations.

Furthermore, the system employs event-driven processing to respond to specific signals and triggers within the clinical trial workflow. For instance, when a new site is added or a new principal investigator is assigned, the Event Processor 130 identifies the necessary documents and creates the appropriate placeholders. This event-driven approach ensures that the eTMF is dynamically updated in real time, reflecting the latest changes and additions. It significantly reduces the lag between an event occurring and the documentation being updated, which is a specific technical improvement over batch processing or manual updates.

The system also uses contextual analysis to interpret the data and signals it receives. For example, it can analyze a study protocol to determine the types of documents required, such as patient consent forms, safety reports, and monitoring visit logs. This contextual interpretation allows the system to make informed decisions about document requirements and organization, ensuring that the eTMF is comprehensive and accurately reflects the study's needs. This capability goes beyond simple data collection, providing a nuanced and intelligent approach to eTMF management.

Moreover, the system is designed to be scalable and flexible, capable of handling eTMFs for studies of varying sizes and complexities. It can adapt to different study designs, regulatory requirements, and document types. This scalability and flexibility ensure that the system can be used across a wide range of clinical trials, from small-scale studies to large, multi-center trials. The ability to scale and adapt is a specific technical feature that enhances the system's utility and applicability, making it suitable for diverse clinical trial scenarios.

User prompts and guidance are another feature of the system. When manual input is required, such as uploading a specific document, the system provides clear prompts and guidance to the user. For example, if a medical license is needed, the system will prompt the user to upload the document and guide them through the process. This user-friendly approach reduces the likelihood of errors and ensures that all necessary documents are collected promptly. The integration of user prompts and guidance is a specific technical feature that enhances the overall user experience and efficiency of the system.

Data security and integrity are also paramount in the autonomous eTMF generation and modification system. The system incorporates robust data security measures to protect sensitive clinical trial information. This includes encryption, access controls, and audit trails to ensure that all data is secure and its integrity is maintained. By ensuring that all data is secure and traceable, the system meets stringent regulatory requirements and provides peace of mind to users. The implementation of advanced security measures is a specific technical improvement that addresses the need for data protection in clinical trials.

Finally, the system is designed to continuously learn and improve from the data it processes. Using machine learning algorithms, it can refine its processes, improve accuracy, and adapt to new types of studies and documents. This continuous learning capability ensures that the system remains up-to-date and capable of handling evolving clinical trial requirements. It represents a specific technical advancement that enhances the system's long-term effectiveness and reliability.

Thus, the autonomous eTMF generation and modification system provides a robust and innovative technical solution to the challenges of managing clinical trial documentation. Through the use of advanced automation techniques, integration of diverse applications, utilization of large language models, event-driven processing, contextual analysis, scalability, user prompts, data security, and continuous learning, the system offers specific technical improvements that enhance the efficiency, accuracy, and reliability of eTMF management. This comprehensive approach ensures that the eTMF is always current, complete, and compliant with regulatory standards, providing a significant advancement over traditional methods. Unlike abstract results or general processes, the disclosed embodiments provide a concrete and detailed implementation that directly addresses technical challenges and offers substantive improvements in the relevant technology.

Aspects of the presently taught approaches may be embodied in the form of a system, a computer program product, or a method. Similarly, aspects of the presently taught approaches may be embodied as hardware, software, or a combination of both. Aspects of the presently taught approaches may be embodied as a computer program product saved on and/or conveyed by one or more computer-readable media in the form of computer-readable program code embodied thereon.

The computer-readable medium may be a computer-readable storage medium. A computer-readable storage medium may be, for example, an electronic, optical, magnetic, electromagnetic, infrared, or semiconductor system, apparatus, or device, or any combination thereof. In some examples a computer-readable storage medium may be termed "non-transitory". The computer-readable medium may also or alternatively be a transmission medium by which instructions may be conveyed. A computer-readable transmission medium may include carrier waves, transmission signals or the like. A computer-readable transmission medium may convey instructions between components of a single computer system and/or between plural separate computer systems.

Computer program code in embodiments of the presently taught approaches may be written in any suitable programming language. The program code may execute on a single computer, or on a plurality of computers. The computer may include a processing unit in communication with a computer-usable medium, where the computer-usable medium contains a set of instructions, and where the processing unit is designed to carry out the set of instructions.

Therefore, from one perspective, there have been described approaches relating to autonomous real-time generation and modification of a dynamic hierarchical file structure for an electronic Trial Master File (eTMF). A method includes receiving, by an event processor, event messages from a message system integrating a plurality of applications, the applications configured to automatically generate messages in real time in response to events. The method further includes determining, by the event processor, for each event message, one or more hierarchical file structure placeholders associated with the corresponding activity based on the metadata of each said event message. The method further includes automatically modifying in real time, by the event processor, the hierarchical file structure to incorporate said one or more hierarchical file structure placeholders. The method further includes storing one or more documents received from the computer system at the first site in said one or more hierarchical file structure placeholders.

Further examples of feature combinations taught by the present disclosure are set out in the following numbered clauses.

Clause 1. A computer-implemented method for autonomous real-time generation and modification of a dynamic hierarchical file structure for an electronic Trial Master File (eTMF), the method comprising: receiving, by an event processor, event messages from a message system integrating a plurality of applications, the applications configured to automatically generate messages in real time in response to events, wherein: each of the events corresponds to a user interaction with one of the applications via a user interface of a computer system at a first site based on an activity associated with the clinical trial, and each event message corresponding to each said user interaction comprises metadata relating to a corresponding activity; determining, by the event processor, for each event message, one or more hierarchical file structure placeholders associated with the corresponding activity based on the metadata of each said event message; automatically modifying in real time, by the event processor, the hierarchical file structure to incorporate said one or more hierarchical file structure placeholders; and storing one or more documents received from the computer system at the first site in said one or more hierarchical file structure placeholders.

Clause 2. The method of clause 1, wherein the metadata relating to the corresponding activity comprises one or more of: a site identifier associated with the site, a milestone identifier corresponding to the activity, and a role designation for a user associated with the activity.

Clause 3. The method of clause 1 or 2, further comprising automatically validating the one or more documents received from the computer system against predefined formats or compliance criteria before storing them in the hierarchical file structure placeholders.

Clause 4. The method of clause 1, 2 or 3, wherein determining, by the event processor, the one or more hierarchical file structure placeholders comprises dynamically applying one or more predefined templates from a template library, the templates specifying expected document types for activities associated with the clinical trial.

Clause 5. The method of any preceding clause, wherein automatically modifying in real time the hierarchical file structure comprises dynamically adapting the structure to fulfill region-specific requirements associated with the first site.

Clause 6. The method of any preceding clause, further comprising tracking the completeness of the hierarchical file structure placeholders across the plurality of sites, and generating real-time quantitative metrics for placeholders that are complete or pending at each site.

Clause 7. The method of any preceding clause, further comprising generating alerts when one or more placeholders remain unfulfilled beyond a predetermined compliance deadline, wherein the alerts are sent to designated users.

Clause 8. The method of any preceding clause, further comprising automatically replacing existing placeholders in the hierarchical file structure with updated placeholders in response to receiving event messages indicating changes to corresponding activities.

Clause 9. The method of any preceding clause, further comprising associating the one or more documents with placeholder-specific metadata, wherein the metadata includes document type, author, version, and applicable event identifier.

Clause 10. The method of any preceding clause, wherein the hierarchical file structure is configured to logically separate placeholders into zones and sections corresponding to study phases, trial sites, or geographic regions.

Clause 11. A system for autonomous real-time generation and modification of a dynamic hierarchical file structure for an electronic Trial Master File (eTMF), the system comprising: a computer having one or more processors in communication with a memory, the memory storing instructions executable by said one or more processors to perform: receiving, by an event processor, event messages from a message system integrating a plurality of applications, the applications configured to automatically generate messages in real time in response to events, wherein: each of the events corresponds to a user interaction with one of the applications via a user interface of a computer system at a first site based on an activity associated with the clinical trial, and each event message corresponding to each said user interaction comprises metadata relating to a corresponding activity; determining, by the event processor, for each event message, one or more hierarchical file structure placeholders associated with the corresponding activity based on the metadata of each said event message; automatically modifying in real time, by the event processor, the hierarchical file structure to incorporate said one or more hierarchical file structure placeholders; and storing one or more documents received from the computer system at the first site in said one or more hierarchical file structure placeholders.

Clause 12. The system of clause 11, wherein the metadata relating to the corresponding activity comprises one or more of: a site identifier associated with the site, a milestone identifier corresponding to the activity, and a role designation for a user associated with the activity.

Clause 13. The system of clause 11 or 12, wherein determining, by the event processor, the one or more hierarchical file structure placeholders comprises dynamically applying one or more predefined templates from a template library, the templates specifying expected document types for activities associated with the clinical trial.

Clause 14. The system of clause 11, 12 or 13, wherein automatically modifying in real time the hierarchical file structure comprises dynamically adapting the structure to fulfill region-specific requirements associated with the first site.

Clause 15. A computer-readable medium comprising instructions that, when executed by one or more processors of a computer, cause said one or more processors to perform a method for autonomous real-time generation and modification of a dynamic hierarchical file structure for an electronic Trial Master File (eTMF), the method comprising: receiving, by an event processor, event messages from a message system integrating a plurality of applications, the applications configured to automatically generate messages in real time in response to events, wherein: each of the events corresponds to a user interaction with one of the applications via a user interface of a computer system at a first clinical trial site based on an activity associated with the clinical trial, and each event message corresponding to each said user interaction comprises metadata relating to a corresponding activity; determining, by the event processor, for each event message, one or more hierarchical file structure placeholders associated with the corresponding activity based on the metadata of each said event message; automatically modifying in real time, by the event processor, the hierarchical file structure to incorporate said one or more hierarchical file structure placeholders; and storing one or more documents received from the computer system at the first clinical trial site in said one or more hierarchical file structure placeholders.

Clause 16. The computer-readable medium of clause 15, wherein the metadata relating to the corresponding activity comprises one or more of: a site identifier associated with the site, a milestone identifier corresponding to the activity, and a role designation for a user associated with the activity.

Clause 17. The computer-readable medium of clause 15 or 16, wherein determining, by the event processor, the one or more hierarchical file structure placeholders comprises dynamically applying one or more predefined templates from a template library, the templates specifying expected document types for activities associated with the clinical trial.

Clause 18. The computer-readable medium of clause 15, 16 or 17, wherein automatically modifying in real time the hierarchical file structure comprises dynamically adapting the structure to fulfill region-specific requirements associated with the first site.

Clause 19. The computer-readable medium of any of clauses 15 to 18, further comprising automatically replacing existing placeholders in the hierarchical file structure with updated placeholders in response to receiving event messages indicating changes to corresponding activities.

Clause 20. The computer-readable medium of any of clauses 15 to 19, wherein the hierarchical file structure is configured to logically separate placeholders into zones and sections corresponding to study phases, trial sites, or geographic regions.

The above discussion is meant to be illustrative of the principles and various embodiments of the presently taught approaches. Numerous variations and modifications will become apparent to those skilled in the art once the above disclosure is fully appreciated. It is intended that the foregoing be interpreted to embrace all such variations and modifications.

## Claims

1. A computer-implemented method for autonomous real-time generation and modification of a dynamic hierarchical file structure for an electronic Trial Master File (eTMF), the method comprising:
receiving, by an event processor, event messages from a message system integrating a plurality of applications, the applications configured to automatically generate messages in real time in response to events, wherein:
each of the events corresponds to a user interaction with one of the applications via a user interface of a computer system at a first site based on an activity associated with the clinical trial, and
each event message corresponding to each said user interaction comprises metadata relating to a corresponding activity;
determining, by the event processor, for each event message, one or more hierarchical file structure placeholders associated with the corresponding activity based on the metadata of each said event message;
automatically modifying in real time, by the event processor, the hierarchical file structure to incorporate said one or more hierarchical file structure placeholders; and
storing one or more documents received from the computer system at the first site in said one or more hierarchical file structure placeholders.

2. The method of claim 1, wherein the metadata relating to the corresponding activity comprises one or more of: a site identifier associated with the site, a milestone identifier corresponding to the activity, and a role designation for a user associated with the activity.

3. The method of claim 1 or 2, further comprising automatically validating the one or more documents received from the computer system against predefined formats or compliance criteria before storing them in the hierarchical file structure placeholders.

4. The method of claim 1, 2 or 3, wherein determining, by the event processor, the one or more hierarchical file structure placeholders comprises dynamically applying one or more predefined templates from a template library, the templates specifying expected document types for activities associated with the clinical trial.

5. The method of any preceding claim, wherein automatically modifying in real time the hierarchical file structure comprises dynamically adapting the structure to fulfill region-specific requirements associated with the first site.

6. The method of any preceding claim, further comprising tracking the completeness of the hierarchical file structure placeholders across the plurality of sites, and generating real-time quantitative metrics for placeholders that are complete or pending at each site.

7. The method of any preceding claim, further comprising generating alerts when one or more placeholders remain unfulfilled beyond a predetermined compliance deadline, wherein the alerts are sent to designated users.

8. The method of any preceding claim, further comprising automatically replacing existing placeholders in the hierarchical file structure with updated placeholders in response to receiving event messages indicating changes to corresponding activities.

9. The method of any preceding claim, further comprising associating the one or more documents with placeholder-specific metadata, wherein the metadata includes document type, author, version, and applicable event identifier.

10. The method of any preceding claim, wherein the hierarchical file structure is configured to logically separate placeholders into zones and sections corresponding to study phases, trial sites, or geographic regions.

11. A system for autonomous real-time generation and modification of a dynamic hierarchical file structure for an electronic Trial Master File (eTMF), the system comprising:
a computer having one or more processors in communication with a memory, the memory storing instructions executable by said one or more processors to perform:
receiving, by an event processor, event messages from a message system integrating a plurality of applications, the applications configured to automatically generate messages in real time in response to events, wherein:
each of the events corresponds to a user interaction with one of the applications via a user interface of a computer system at a first site based on an activity associated with the clinical trial, and
each event message corresponding to each said user interaction comprises metadata relating to a corresponding activity;
determining, by the event processor, for each event message, one or more hierarchical file structure placeholders associated with the corresponding activity based on the metadata of each said event message;
automatically modifying in real time, by the event processor, the hierarchical file structure to incorporate said one or more hierarchical file structure placeholders; and
storing one or more documents received from the computer system at the first site in said one or more hierarchical file structure placeholders.

12. The system of claim 11, wherein the metadata relating to the corresponding activity comprises one or more of: a site identifier associated with the site, a milestone identifier corresponding to the activity, and a role designation for a user associated with the activity.

13. The system of claim 11 or 12, wherein determining, by the event processor, the one or more hierarchical file structure placeholders comprises dynamically applying one or more predefined templates from a template library, the templates specifying expected document types for activities associated with the clinical trial.

14. The system of claim 11, 12 or 13, wherein automatically modifying in real time the hierarchical file structure comprises dynamically adapting the structure to fulfill region-specific requirements associated with the first site.

15. A computer-readable medium comprising instructions that, when executed by one or more processors of a computer, cause said one or more processors to perform then method of any of claims 1 to 10.
